# EUROPÄISCHE PATENTSCHRIFT

(11) **EP 0 263 385 B1**
(45) Veröffentlichungstag und Bekanntmachung des Hinweises auf die Patenterteilung: **16.06.1993**
(21) Anmeldenummer: 87114098.4
(22) Anmeldetag: 26.09.1987
(51) Int. Cl.: C07C 45/38, C07C 45/39, C07C 47/21, C07C 49/203

(54) **Verfahren zur kontinuierlichen Herstellung von Aldehyden und Ketonen**
Process for the continuous preparation of aldehydes and ketones
Procédé de préparation en continu d'aldéhydes et de cétones

(30) Priorität: 04.10.1986 DE 3633885
(43) Veröffentlichungstag der Anmeldung: 13.04.1988
(73) Patentinhaber: BASF Aktiengesellschaft, 67063 Ludwigshafen (DE)
(72) Erfinder: Brenner, Karl, D-6700 Ludwigshafen (DE); Eggersdorfer, Manfred, Dr., D-6710 Frankenthal (DE); Ruppel, Wilhelm, Dr., D-6830 Schwetzingen (DE); Schultheiss, Harald, Dr., D-6710 Frankenthal (DE); Scholz, Hans-Ulrich, Dr., D-6719 Weisenheim (DE)

(56) Entgegenhaltungen:
- EP-A- 0 217 280
- EP-A- 0 218 124
- DE-A- 3 612 213
- FR-A- 2 194 669
- US-A- 4 359 587

## Beschreibung

Die vorliegende Erfindung betrifft ein neues Verfahren zur kontinuierlichen Herstellung von Aldehyden und Ketonen der allgemeinen Formel I 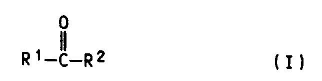
in der R¹ Wasserstoff oder einen organischen Rest mit 1 bis n C-Atomen und R² einen nicht-aromatischen organischen Rest mit 1 bis m C-Atomen bedeutet, wobei (m+n) einen Wert von 2 bis 24 hat und wobei R¹ und R² auch zu einem 4- bis 12-gliedrigen Ring verbunden sein können, durch Oxidation eines Alkohols der allgemeinen Formel II 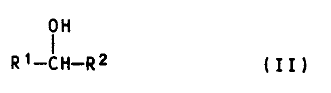
mit Sauerstoff oder Sauerstoff enthaltenden Gasen bei erhöhter Temperatur in der Gasphase in Gegenwart eines Katalysators.

Es ist aus der US-A 2 042 220 bekannt, daß man 3-Methyl-3-buten-1-ol mit einem Überschuß an Sauerstoff bei 360 bis 550°C in Gegenwart von Metallkatalysatoren, z.B. Kupfer- und Silberkatalysatoren, zu 3-Methyl-3-buten-1-al oxidieren kann. Die Katalysatoren können Legierungen, Metallverbindungen oder elementares Metall sein. Bevorzugt sind aktivierte Katalysatoren; als Aktivierungsoperationen werden eine Oberflächenamalgamierung des Metalls und anschließendes Erhitzen der Metalloberfläche angegeben. Die Herstellung von Kupfer- und Silberkatalysatoren in den Beispielen besteht in der Reduktion von Kupferoxidpartikeln bei 300°C unter Wasserstoffatmosphäre oder in Amalgamierung und Erhitzen von Silberdrahtnetzen. Außerdem ist der DE-B-20 41 976 zu entnehmen, daß bei dem bekannten Verfahren erhebliche Mengen an unerwünschten Nebenprodukten gebildet werden.

Die DE-A-2 517 859 beschreibt die Dehydrierung ungesättigter Alkohole an einem Kupferkatalysator, der eine spezifische Oberfläche von 0,01 bis 1,5 m²/g hat, im wesentlichen in Abwesenheit von Sauerstoff bei 150 bis 300°C. Im Falle von α,β-ungesättigten Alkoholen als Ausgangsstoffen werden β,γ-ungesättigte Aldehyde und gesättigte Aldehyde als Nebenprodukte gebildet; die Selektivität für α,β-ungesättigte Aldehyde ist gering (Seite 2, letzter Absatz). Solche Gemische müssen in aufwendigen Trennoperationen in ihre Komponenten zerlegt werden.

In der DE-B-20 20 865 und der DE-B-20 41 976 wird die Dehydrierung von β,γ-ungesättigten Alkoholen bzw. α,β-ungesättigten Alkoholen zu α,β-ungesättigten Aldehyden beschrieben. Als Dehydrierungskatalysatoren werden auch Mischkatalysatoren, z.B. solche aus Kupfer und Silber genannt. Nachteilig ist jedoch, daß man erhebliche Mengen an nukleophilen Substanzen zusetzen muß. Im Falle der Umsetzung von 3-Methyl-3-buten-1-ol werden gute Ergebnisse nur bei unvollständigen Umsätzen erhalten, was nach der Lehre der DE-B-22 43 810 zu Schwierigkeiten bei der Abtrennung des unumgesetzten Ausgangsstoffes führt.

Dehydriert man 3-Methyl-3-buten-1-ol nach dem Verfahren der DE-B-25 17 859 ohne Zusatz von Sauerstoff an metallischem Kupfer, so entstehen erhebliche Mengen an Isovaleraldehyd und die Aktivität der Katalysatoren fällt innerhalb weniger Tage rasch ab, so daß häufig regeneriert werden muß.

In der FR-A-2 231 650 wird die Herstellung von Aldehyden und Ketonen aus den entsprechenden Alkoholen durch Luftoxidation bei 250 - 600°C in Gegenwart eines Gold-Katalysators beschrieben. Der Vorteil des Gold-Katalysators liegt in der höheren Selektivität im Vergleich zu Kupfer und Silber-Katalysatoren, sodaß die Nebenprodukt-Bildung verringert wird. Nachteilig bei diesem Verfahren sind die hohen Katalysatorkosten, da ein massiver Gold-Kontakt Verwendung findet.

In der DE-B-27 15 209 und der EP-B-55 354 wird die oxidative Dehydrierung von 3-Alkyl-buten-1-olen an Katalysatoren, die aus Schichten von Silber- und/oder Kupferkristallen bestehen, unter Zusatz von molekularem Sauerstoff beschrieben. Die Sauerstoffmengen liegen im Bereich von 0,3 bis 0,7 mol, bezogen auf den Einsatzstoff. Nachteilig bei diesem Verfahren ist, daß hohe Katalysatorkosten durch Verwendung von massivem Silber auftreten und gute Selektivitäten nur erzielt werden können, wenn definierte Katalysatorkorngrößen bzw. -korngrößenverteilungen in einem Schichtenaufbau, unter Umständen sogar bestimmte Mischungen von Schichten aus Kupfer- und Silberkristallen eingesetzt werden. Dies bedeutet sowohl eine aufwendige Füllung des Reaktors als auch eine aufwendige Katalysatorrückgewinnung. Zudem tritt bei den dabei angewandten hohen Reaktionstemperaturen Sinterung der Metallkristalle auf, was zu Druckanstieg und geringen Standzeiten führt.

In der JP-A-60/246340 wird die Gasphasenoxidation von 3-Methyl-2-buten-1-ol zu 3-Methyl-2-buten-1-al bei 300 - 600°C in Gegenwart von Sauerstoff und eines Trägerkatalysators beschrieben. Der Trägerkatalysator ist in komplizierter Weise durch Tränken des Trägers mit wäßrigen Lösungen von AgNO₃, Cu(NO₃)₂ x 3 H₂O und Mg(NO₃)₂ x 6 H₂O, Trocknen, Calcinieren in einem bestimmten Temperaturbereich sowie Aktivieren unter Wasserstoff-Atmosphäre herzustellen. Der Katalysator liefert zwar eine gute Selektivität von 96,6 %, jedoch nur unter Inkaufnahme von geringem Umsatz, so daß er für technische Zwecke kaum in Betracht kommt.

In der JP-A-58/059 933 wird die Herstellung von Aldehyden und Ketonen durch oxidative Dehydrierung von Alkoholen in Gegenwart eines Silberkatalysators, der zusätzlich Phosphor enthält, beschrieben. Um die Selektivität der Umsetzung aufrecht zu erhalten, wird in den Alkoholstrom zusätzlich eine Phosphorverbindung eingebracht, so daß man eine Verunreinigung des Produktes in Kauf nehmen muß. In Anbetracht der Verwendung der Aldehyde für Riechstoffe und Vitamine ist der Zusatz einer phosphororganischen Verbindung evident nachteilig.

In US-A 4 359 587 wird zur Oxidation von Alkoholen zu den entsprechenden Aldehyden ein silberhaltiger Trägerkatalysator mit einem speziellen Trägermaterial, bestehend aus einer genau definierten Mischung aus Aluminiumoxid mit Cristobalit, verwendet. Als Folge seiner Herstellungsweise ist dieser Katalysator unregelmäßig geformt und porös.

Gemäß FR-A 2 194 664 werden zur Herstellung von Aldehyden aus den entsprechenden Alkoholen die katalytisch aktiven Metalle vorzugsweise in Form von Metallgazen eingesetzt. In Beispiel II dieser Schrift wird ein Kupfer auf Alundum-Katalysator hergestellt, der außerdem noch Barium enthält. Aus seiner Herstellungsweise muß geschlossen werden, daß es sich bei diesem Kupferkatalysator nicht um einen metallischen, sondern um einen oxidischen Kupferkatalyator handelt, der porös ist. Das Verhältnis von Katalysatordurchmesser zu Rohrdurchmesser beträgt gemäß Beispiel III dieser Schrift 0,4.

Da alle diese bekannten Verfahren zur Herstellung von Aldehyden und Ketonen im Hinblick auf einen unkomplizierten und wirtschaftlichen Betrieb, eine lange Katalysatorstandzeit und die Reinheit der Reaktorausträge nicht befriedigten, lag der Erfindung die Aufgabe zugrunde, diesen Nachteilen abzuhelfen.

Demgemäß wurde ein Verfahren zur kontinuierlichen Herstellung von Aldehyden und Ketonen der allgemeinen Formel I 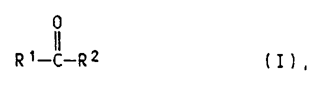
in der R¹ Wasserstoff oder einen organischen Rest mit 1 bis n C-Atomen und R² einen nicht-aromatischen organischen Rest mit 1 bis m C-Atomen bedeutet, wobei (m+n) einen Wert von 2 bis 24 hat und wobei R¹ und R² auch zu einem 4- bis 12-gliedrigen Ring verbunden sein können, durch Oxidation eines Alkohols der allgemeinen Formel II 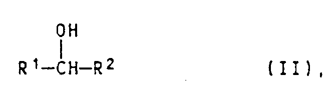
mit Sauerstoff oder Sauerstoff enthaltenden Gasen bei erhöhter Temperatur in der Gasphase in Gegenwart eines Katalysators gefunden, welches dadurch gekennzeichnet ist, daß man die Oxidation mittels eines Trägerkatalysators aus Kugeln eines inerten Trägermaterials, die mit 0,1 bis 20 Gew.-%, bezogen auf die Menge des Trägers, einer Schicht aus metallischem Kupfer, Silber und/oder Gold in Form einer glatten, abriebfesten Schale beschichtet sind, in einem Rohrreaktor oder Rohrbündelreaktor vornimmt, wobei der Innendurchmesser D des Rohres bzw. der Rohre 10 bis 50 mm beträgt und wobei für den größten Durchmesser d der beschichteten Trägerkatalysatoren die Beziehung d = 0,1 bis 0,2 gilt.

Gegebenenfalls kann der Katalysator auch mit einem inerten, nicht mit aktiver Masse beschichteten Material verdünnt werden. Als inerte Materialien, die auch als Trägermaterial geeignet sind, kommen keramische Massen, wie Aluminiumoxid, Siliciumdioxid, Magnesiumoxid, Siliciumcarbid oder vor allem Steatit in Frage. Eine Kontaktschicht sollte aber mindestens 10 % an Teilchen mit aktiver Masse enthalten.

Als inerte Formkörper für den Katalysator eignen sich in erster Linie Kugeln sowie daneben auch sonstige Körper wie Ellipsoide, Zylinder oder Ringe. Der Durchmesser d der Kugeln bzw. der größte Durchmesser der sonstigen Körper kann im Bereich von 1 bis 10 mm Durchmesser liegen, wobei sich die Durchmesser definitionsgemäß nach dem inneren Durchmesser der Reaktorrohre richten.

Das als Katalysator aktive Metall wird vorzugsweise durch Flammspritzen auf das Inertmaterial aufgebracht, jedoch kommen auch andere Methoden z.B. Tränken oder Plasmaspritzen in Betracht, sofern dabei eine abriebfeste Schale entsteht, die im übrigen möglichst glatt sein sollte.

Im Vergleich zum Stand der Technik liefert das erfindungsgemäße Verfahren überraschend auf einfacherem und wirtschaftlicherem Wege ein besseres Gesamtergebnis in Bezug auf Ausbeute, Raum-Zeit-Ausbeute und Reinheit des Endstoffes. Insbesondere ist die Menge an Produkt zum eingesetzten Katalysator sehr viel größer, so daß bei Verwendung der selektiven Edelmetall-Katalysatoren Silber oder auch Gold ein wirtschaftliches Verfahren realisiert werden kann. Der Katalysator ist einfach herzustellen und erlaubt besonders im Falle von Kugeln eine einfache Befüllung des Reaktors. Ein weiterer Vorteil der regelmäßigen Form des Katalysators ist, daß ohne weitere Maßnahmen eine geordnete dichtere Packung im Reaktor erzielt wird und bei Rohrbündelreaktoren jedes Einzelrohr aufgrund der gleichmäßigen Schüttung ähnlichen Druckverlust zeigt. Der in den vielen Röhren eines Rohrbündelreaktors gleiche Druckverlust führt zu gleicher Anströmung der einzelnen Röhren und dadurch offenbar zu einer wesentlichen Verbesserung der Selektivität der Umsetzung. Im Verlauf der Reaktion werden einzelne Rohre nicht stärker belastet, sodaß die Standzeit des Katalysators sehr hoch ist und in der Praxis bei mehreren Monaten liegt.

Man führt das Verfahren so durch, daß man die Alkohole II in die Gasphase überführt und gemeinsam mit Sauerstoff bei Temperaturen von 300 bis 600°C, vorzugsweise 350 bis 450°C, über den Katalysator leitet.

Als oxidierendes Agens lassen sich sowohl der reine Sauerstoff als auch freien Sauerstoff enthaltende Gase, insbesondere Luft, verwenden. Sauerstoff und der Alkohol II werden zweckmäßig im Molverhältnis von 0,2 bis 0,6, insbesondere von 0,3 bis 0,5 Mol Sauerstoff je Mol Alkohol angewandt. Man verwendet gegebenenfalls unter den Reaktionsbedingungen inerte Lösungsmittel, z.B. Wasser oder Ether. Es ist ein Vorteil des erfindungsgemäßen Verfahren, daß neben dem durch die Luft eingetragenen Stickstoff kein weiteres Inertgas verwendet werden muß. Dies bedeutet im Vergleich zu den vorbeschriebenen Verfahren eine Reduzierung des Inertgasanteils und damit eine Vereinfachung der Aufarbeitung, weil das Wertprodukt aus einem geringeren Gesamtgasstrom kondensiert werden kann.

Vorzugsweise verwendet man einen Rohrbündelreaktor mit 100 bis 10000 Rohren einer Rohrlänge von 10 bis 30 cm. Für Versuchszwecke genügt die Verwendung eines Rohres.

Zweckmäßig belastet man den Katalysator mit 0,5 bis 4 t, insbesondere 0,7 bis 2 t Ausgangsstoff II je m² Katalysatorbettquerschnitt und Stunde.

Das Reaktionsgemisch arbeitet man in an sich bekannter Weise auf. Beispielsweise absorbiert man die heißen Reaktionsgase mit einem Lösungsmittel wie Wasser oder Dimethylformamid oder vorzugsweise im kondensiertem Produktgemisch.

Als Ausgangsverbindungen kommen prinzipiell beliebige primäre Alkohole II in Betracht. Das gleiche gilt für sekundäre Alkohole, sofern nicht beide Reste R¹ und R² für aromatische Reste stehen. Die Reste R¹ und R² können ihrerseits inerte Substituenten wie Fluor, Chlor, Brom, die Cyangruppe und tertiäre Aminogruppen tragen und durch Sauerstoff oder sauerstoffhaltige Gruppierungen wie -CO- und -O-CO- unterbrochen sein.

Genannt seien:
- gesättigte aliphatische primäre Alkohole mit 1 bis 24, vorzugsweise 6 bis 18 C-Atomen, wobei hauptsächlich verzweigte Alkohole und Etheralkohole wie 2-Ethoxyethanol in Betracht kommen;
- besonders bevorzugt ungesättigte aliphatische primäre Alkohole mit 3 bis 24, vorzugsweise 4 bis 12 C-Atomen, deren Doppelbindung oder Doppelbindungen nicht in 1,2-Stellung liegen, z.B.
   But-2-enol
   But-3-enol
   Pent-3-enol
   Pent-4-enol
   Hex-3-enol
   Hex-4-enol
   Hex-5-enol
   Hept-3-enol
   Hept-4-enol
   Hept-5-enol
   Oct-4-enol
   Oct-5-enol
   sowie besonders die C₁-C₄-Alkylhomologen, besonders die Methylhomologen dieser Alkohole, darunter vor allem die Butenole IIa und IIb 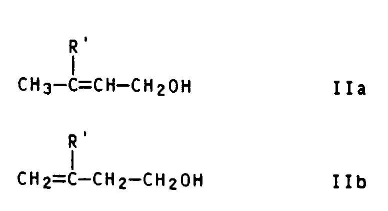 in denen Rʹ eine C₁-C₄-Alkylgruppe, besonders die Methylgruppe bedeutet. Allgemein eignet sich das Verfahren vor allem für Alk-2-enole, da sich hierbei eine Gruppierung mit -CH=CH-(C=O)-Konjugation ausbildet, wodurch die Dehydrierung energetisch begünstigt wird;
- cycloaliphatisch-aliphatische primäre Alkohole wie Hydroxymethylcyclohexan, Hydroxymethylcyclohex-1-en und die Hydroxymethyltetrahydrofurane;
- araliphatische primäre Alkohole wie 2-Phenylethanol;
- sekundäre aliphatische Alkohole mit 3 bis 24, vorzugsweise 4 bis 12 C-Atomen, beispielsweise
   Butan-2-ol
   3-Methylbutan-2-ol
   But-3-en-2-ol
   3-Methylbut-3-en-2-ol
   Pentan-2-ol
   Pent-3-en-2-ol
   Pent-4-en-2-ol
   Pentan-3-ol
   Pent-1-en-3-ol
- sekundäre araliphatische Alkohole wie 1-Phenylethanol, 1-Phenylpropanol und 1-Phenylprop-2-enol;
- cyclische sekundäre Alkohole, insbesondere solche mit 5 bis 6 Ringgliedern wie Cyclohexanol, 3-Hydroxytetrahydrofuran, 3-Hydroxytetrahydropyran und 4-Hydroxytetrahydropyran.

Die Alkohole II sind entweder bekannt oder nach bekannten Verfahren erhältlich.

Die nach dem Verfahren der Erfindung herstellbaren Carbonylverbindungen I sind wertvolle Ausgangsstoffe für die Herstellung von Farbstoffen, Schädlingsbekämpfungsmitteln, Pharmazeutika, Kunststoffen, Riechstoffen wie Citral, Vitaminen, wie den Vitaminen A und E und von Chrysanthemumsäure.

Das neue Verfahren zeichnet sich durch große Wirtschaftlichkeit aus. Im allgemeinen gestattet es Katalysatorbelastungen von 50 bis 500 mol pro Stunde pro l Kontaktfüllung.

### Herstellung des Katalysators

0,5 l Kugeln aus Steatit mit einem Durchmesser von 1,6 bis 2,0 mm wurden in eine offene Drehtrommel gegeben und mit Silberpulver der Korngröße 16 Mikrometer nach der Methode des Flammspritzens beschichtet. Dabei wurde das Silberpulver in eine Sauerstoff/Acetylen-Flamme eingebracht und mit den Flammgasen in teilweise geschmolzener Form auf den Träger transportiert. Die Pulvermenge wurde so bemessen, daß der fertige Katalysator etwa 4 Gew.% Silber, bezogen auf die Gesamtmasse enthielt.

Während der Beschichtung wurde der Träger in der Drehtrommel mit 30 Umdrehungen pro Minute umgewälzt.

Die Temperaturen im zu beschichtenden Gut betrugen etwa 600°C und die Beschichtung nahm etwa 30 Minuten in Anspruch.

Nach Beendigung des Flammspritzens erhielt man Katalysatorkugeln mit geschlossener glatter Schale aus metallischem Silber.

### Beispiel 1

### Herstellung von 3-Methyl-3-but-3-enal mit einem Silberkatalysator

11 ml des Katalysators wurden in ein Reaktorrohr von 10 cm Länge und 13 mm Innendurchmesser eingefüllt. Durch das Rohr wurde dann bei einer Temperatur von 390°C und einem Druck von 1,1 bar ein Gemisch aus 30,1 Nl/h technischem 3-Methyl-3-buten-1-ol (Wassergehalt rd. 12 - 15 Gew.%) und 45,9 Nl/h Luft geleitet. Die Sauerstoffmenge betrug dabei 0,32 mol pro Mol 3-Methyl-3-butenol. Die Reaktionsgase wurden aufgefangen und analysiert. Bei einem Alkoholumsatz von 73 % betrug die Selektivität 89 %; dieses Ergebnis blieb über die Versuchsdauer von 10 Tagen unverändert.

### Beispiel 2

### Herstellung von 3-Methylbut-3-enol mit einem Kupferkatalysator

50 ml eines Katalysators (Steatitkugeln mit 3,5 - 4,5 mm Durchmesser, Schicht aus Kupfer mit einem Gewichtsanteil von 9,57 % bezogen auf Inertmaterial, der wie der Silberkatalysator hergestellt worden war) wurden in einem Reaktorrohr mit einem Innendurchmesser von 22 mm und 20 cm Länge bei einer Reaktionstemperatur von 390°C und einem Druck von 1,1 bar mit einem Gemisch aus 27,7 Nl/h 3-Methyl-3-buten-1-ol und 75 Nl/h Luft beschickt. Aus einem Teilstrom des Reaktionsgases wurden die Reaktionsprodukte aufgefangen und analysiert. Es wurde ein Umsatz von 78,6 % und eine Aldehyd-Selektivität von 78,1 % erzielt.

Formaldehyd und Säuren waren nur in Spuren entstanden. Diese Ergebnisse wurden nach einer Gesamtbetriebszeit des Katalysators von 9 Tagen erzielt. Auch nach einer 4-wöchigen Betriebszeit war kein Abfall von Aktivität und Selektivität festzustellen.

### Beispiel 3

### Herstellung von Oct-1-en-3-on mit einem Silberkatalysator

Analog Beispiel 1 wurden 22,5 Nl/h Oct-1-en-3-ol und 45,2 Nl/h Luft bei 380°C umgesetzt. Der Alkoholumsatz betrug 90 % und die Selektivität bezüglich des Ketons 82 %.

## Patentansprüche

1. Verfahren zur kontinuierlichen Herstellung von Aldehyden und Ketonen der allgemeinen Formel I 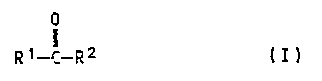 in der R¹ Wasserstoff oder einen organischen Rest mit 1 bis n C-Atomen und R² einen nicht-aromatischen organischen Rest mit 1 bis m C-Atomen bedeutet, wobei (m+n) einen Wert von 2 bis 24 hat und wobei R¹ und R² auch zu einem 4- bis 12-gliedrigen Ring verbunden sein können, durch Oxidation eines Alkohols der allgemeinen Formel II 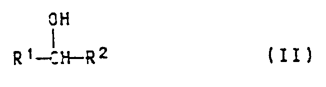 mit Sauerstoff oder Sauerstoff enthaltenden Gasen bei erhöhter Temperatur in der Gasphase in Gegenwart eines Katalysators, dadurch gekennzeichnet, daß man die Oxidation mittels eines Trägerkatalysators aus Kugeln eines inerten Trägermaterials, die mit 0,1 bis 20 Gew.-%, bezogen auf die Menge des Trägers, einer Schicht aus metallischem Kupfer, Silber und/oder Gold in Form einer glatten, abriebfesten Schale beschichtet sind, in einem Rohrreaktor oder Rohrbündelreaktor vornimmt, wobei der Innendurchmesser D des Rohres bzw. der Rohre 10 bis 50 mm beträgt und wobei für den größten Durchmesser d der beschichteten Trägerkatalysatoren die Beziehung d = 0,1 bis 0,2 D gilt.

2. Verfahren nach den Ansprüchen 1, dadurch gekennzeichnet, daß das Trägermaterial aus Steatit besteht.

3. Verfahren nach den Ansprüchen 1 und 2, dadurch gekennzeichnet, daß die aktive Masse des Trägerkatalysators aus Kupfer oder Silber besteht.

4. Verfahren nach den Ansprüchen 1 bis 3, dadurch gekennzeichnet, daß man die Oxidation bei 350 bis 450°C vornimmt.

5. Verfahren nach den Ansprüchen 1 bis 4, dadurch gekennzeichnet, daß man es auf die Oxidation von Alkoholen der allgemeinen Formeln IIa und IIb 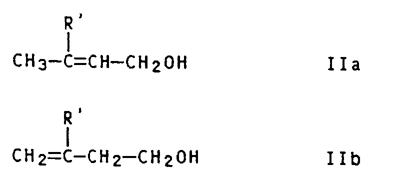 in denen Rʹ für eine C₁-C₄-Alkylgruppe steht, anwendet.

## Claims

1. A process for the continuous preparation of an aldehyde or ketone of the general formula I 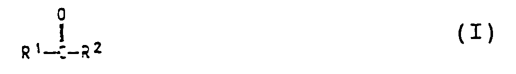 where R¹ is hydrogen or an organic radical of 1 to n carbon atoms and R² is a non-aromatic organic radical of 1 to m carbon atoms, (m+n) ranging from 2 to 24 and R¹ and R² being combinable to form a 4- to 12-membered ring, by oxidizing an alcohol of the general formula II 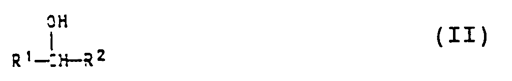 with oxygen or an oxygen-containing gas at elevated temperatures in the gas phase in the presence of a catalyst, which comprises effecting the oxidation by means of a supported catalyst composed of balls of an inert carrier material coated with from 0.1 to 20% by weight, based on the amount of carrier, of a layer of metallic copper, silver and/or gold in the form of a smooth, abrasion-resistant shell in a tubular reactor or tube bundle reactor where the internal diameter D of the tube or tubes ranges from 10 to 50 mm and the largest diameter d of the coated supported catalysts is subject to the relationship d = from 0.1 to 0.2 D.

2. A process as claimed in claim 1, wherein the carrier material is made of steatite.

3. A process as claimed in claim 1 or 2, wherein the active material of the supported catalyst is made of copper or silver.

4. A process as claimed in any of claims 1 to 3, wherein the oxidation is carried out at from 350 to 450°C.

5. A process as claimed in any of claims 1 to 4, which is applied to the oxidation of an alcohol of the general formula IIa or IIb 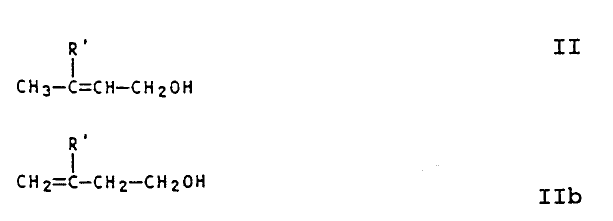 where R' is C₁-C₄-alkyl.

## Revendications

1. Procédé de préparation en continu d'aldéhydes et de cétones de formule générale I 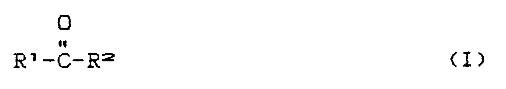 dans laquelle R¹ représente un atome d'hydrogène ou un reste organique renfermant 1 à n atomes de carbone et R² représente un reste organique non aromatique renfermant 1 à m atomes de carbone, (m+n) ayant une valeur de 2 à 24 et R¹ et R² pouvant également être reliés en un noyau à 4-12 maillons, par oxydation d'un alcool de formule générale II 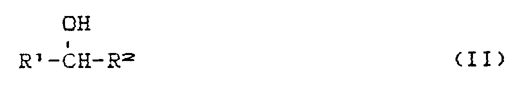 par de l'oxygène ou des gaz contenant de l'oxygène, à température élevée, en phase gazeuse et en présence d'un catalyseur, caractérisé en ce qu'on effectue l'oxydation dans un réacteur tubulaire ou un réacteur à faisceau de tubes, au moyen d'un catalyseur sur support constitué par des billes d'une matière inerte de support qui sont revêtues de 0,1 à 20% en poids, par rapport à la quantité du support, d'une couche de cuivre, d'argent et/ou d'or métallique sous forme d'une coquille lisse et résistante à l'abrasion, le diamètre intérieur D du tube ou des tubes étant compris entre 10 et 50 mm, et la relation d = 0,1 à O,2 D s'appliquant au plus grand diamètre d des billes du catalyseur sur support revêtu.

2. Procédé selon la revendication 1, caractérisé en ce que la matière de support est constituée par de la stéatite.

3. Procédé selon la revendication 1 ou 2, caractérisé en ce que la masse active du catalyseur sur support est faite de cuivre ou d'argent.

4. Procédé selon l'une quelconque des revendications 1 à 3, caractérisé en ce que l'oxydation est effectuée à une température de 350 à 450°C.

5. Procédé selon l'une quelconque des revendications 1 à 4, caractérisé en ce qu'on l'applique à l'oxydation d'alcools de formules générales IIa et IIb 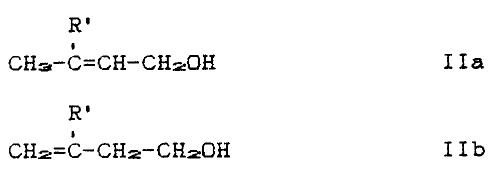 dans lesquelles R' est mis pour un groupement alkyle en C₁-C₄.
